# DEMANDE DE BREVET EUROPEEN

(11) **EP 1 008 338 A1**
(43) Date de publication de la demande: **14.06.2000**
(21) Numéro de dépôt: 99403074.0
(22) Date de dépôt: 08.12.1999
(51) Int. Cl.: A61K 7/021, A61K 7/02, A61K 7/13, A61K 7/00

(54) **Utilisation d'un composé indigoîde dans une composition cosmétique, notamment de maquillage, pour lui conférer des propriétés anti-microbiennes et de longue tenue dans le temps**

(30) Priorité: 11.12.1998 FR 9815693
(71) Demandeur: L'OREAL, 75008 Paris (FR)
(72) Inventeur: Lemann, Patricia, 94000 Creteil (FR)
(74) Mandataire: Casalonga, Axel

(57) **Abrégé**

L'invention concerne l'utilisation d'au moins un composé de formule : 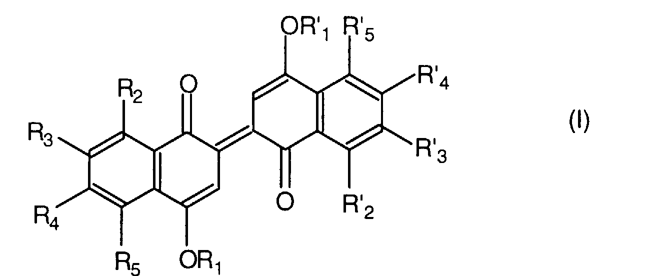 dans laquelle R₁ et R'₁ sont des radicaux alkyles en C₁-C₁₈, éventuellement substitués par un ou plusieurs halogènes, hydroxyles et/ou interrompus par un ou plusieurs hétéroatomes, et R₂ à R₅ et R'₂ à R'₅ sont des atomes d'hydrogène, d'halogène, des radicaux hydroxyles, alkyles, alkyloxy, acyles ou acyloxy en C₁-C₆, dans une composition cosmétique, notamment de maquillage, pour lui conférer des propriétés anti-microbiennes et de longue tenue dans le temps et/ou de non-migration et/ou de sans-transfert et/ou de non-formation de stries.

L'invention concerne également une composition cosmétique, notamment de maquillage et en particulier un fard à paupières, contenant un tel composé dans un liant siliconé.

## Description

La présente invention a pour objet l'utilisation d'un composé indigoïde dans une composition cosmétique, notamment de maquillage, pour lui conférer des propriétés anti-microbiennes et de longue tenue dans le temps et/ou de non-migration et/ou de sans-transfert et/ou de non-formation de stries, ainsi qu'une composition cosmétique, notamment de maquillage, contenant un tel composé indigoïde en association avec un liant siliconé.

Les compositions cosmétiques, et notamment les compositions de maquillage telles que les poudres libres ou compactes, les fonds de teint, les fards à joues ou à paupières, les rouges à lèvres ou les vernis à ongles, sont constituées d'un véhicule approprié et de différents agents colorants destinés à conférer une certaine couleur auxdites compositions avant et/ou après leur application sur la peau, les muqueuses comme la partie interne des paupières inférieures, les semi-muqueuses telles que les lèvres et/ou les phanères telles que les ongles, les cils, les sourcils ou les cheveux.

Pour créer des couleurs, on utilise aujourd'hui une gamme d'agents colorants assez limitée, parmi lesquels on peut citer des composés généralement insolubles dans les milieux aqueux et organiques tels que des laques organiques, des pigments minéraux ou des pigments nacrés.

Les pigments et laques utilisés dans le domaine du maquillage sont d'origine et de nature chimique très diverses. Leurs propriétés physico-chimiques, notamment granulométrie, surface spécifique, densité, etc., sont donc très différentes. Ces différences se traduisent par des variations de comportement : leur facilité de mise en oeuvre, de dispersion dans le milieu; leur stabilité à la lumière, à la température; leurs propriétés mécaniques; leur tenue dans le temps; leur pouvoir colorant et leur pouvoir couvrant.

Ainsi, les pigments minéraux, en particulier les oxydes minéraux tels que les oxydes de fer, le bleu d'Outremer, le bleu de Prusse (ferrocyanure ferrique), le bleu de cobalt (CoOAl₂O₃) ou le violet de manganèse, sont très stables à la lumière et au pH, mais donnent des couleurs plutôt ternes, fades et pâles, et/ou de faible pouvoir colorant, les couleurs étant facilement diluées lorsqu'on les mélange avec un pigment blanc du type dioxyde de titane et/ou de faible pouvoir couvrant, c'est-à-dire transparentes à l'application. Il est donc nécessaire d'en introduire une grande quantité dans les formulations cosmétiques pour obtenir un trait suffisamment saturé. Ce fort pourcentage de particules minérales peut néanmoins affecter la brillance de la composition, son homogénéité à l'application, sa tenue et son confort. Pour le violet de manganèse, on observe en outre l'apparition de moisissures à la surface de certaines compositions.

Pour obtenir des effets colorés, on peut encore employer des pigments nacrés de couleurs variées, mais jamais très intenses, qui permettent d'obtenir des effets irisés mais le plus souvent assez faibles.

Dans le domaine de la coloration capillaire temporaire ou fugace, qui donne lieu à une modification légère de la couleur naturelle de la chevelure qui tient d'un shampooing à l'autre et qui sert à embellir ou corriger une nuance déjà obtenue, on a déjà proposé une coloration avec des pigments minéraux usuels pour apporter un reflet temporaire aux cheveux, mais les nuances obtenues par cette coloration restent assez ternes, trop uniformes et peu ludiques.

Dans le domaine du maquillage, seules les laques organiques permettaient jusqu'à présent d'obtenir des couleurs vives et intenses. Cependant, la plupart des laques organiques présentent une très mauvaise tenue à la lumière, qui se traduit par une atténuation très nette de leur couleur dans le temps, sont très transparentes à l'application, c'est-à-dire peu couvrantes et ont un faible pouvoir colorant. Elles peuvent également être instables à la température et/ou au pH. De plus, certaines laques génèrent un dégorgement trop important, c'est-à-dire qu'elles présentent l'inconvénient de tacher le support sur lequel elles sont appliquées. Ainsi, ceci peut avoir pour conséquence de tâcher les lentilles oculaires dans le cas des eye-liners ou des mascaras, ou de laisser une coloration sur la peau ou les ongles après démaquillage dans le cas des rouges à lèvres ou des vernis à ongles. Enfin, l'instabilité des laques est encore aggravée lorsqu'elles sont associées à des pigments photoréactifs comme le dioxyde de titane. Or, ces pigments sont très largement utilisés dans le maquillage, notamment pour la protection contre le rayonnement UV. Par conséquent, l'utilisation des laques organiques en cosmétique est assez limitée, ce qui a pour conséquence une limitation des teintes réalisables.

Ainsi, il subsiste le besoin de disposer d'agents colorants susceptibles d'être utilisés en cosmétique et permettant d'obtenir une coloration adéquate des compositions et du film de maquillage obtenu, lesdits agents colorants ne devant pas migrer ni dégorger sur le support sur lequel lesdites compositions sont déposées, ou transférer sur un autre support, devant posséder un pouvoir colorant et un pouvoir couvrant élevés, une longue tenue dans le temps, sans apparition de stries, en particulier sur les paupières. De plus, ces agents colorants ne doivent pas favoriser le développement de moisissures.

Après de nombreuses recherches, la demanderesse a mis en évidence que l'utilisation d'une famille bien précise de composés organiques permettait d'obtenir un tel résultat.

Ainsi, l'invention a pour objet l'utilisation d'au moins un composé indigoïde de formule (I) : 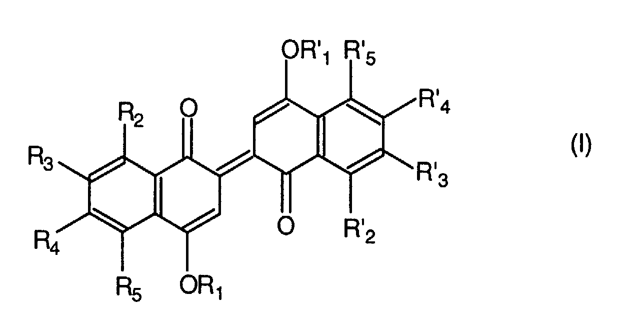 dans laquelle :
- R₁ et R'₁ sont, indépendamment l'un de l'autre, des radicaux alkyles, linéaires, ramifiés ou cycliques, saturés ou insaturés, ayant 1 à 18 atomes de carbone, et éventuellement substitués par un ou plusieurs halogènes, et/ou par un ou plusieurs radicaux hydroxyles, et/ou interrompus par un ou plusieurs hétéroatomes;
- R₂, R'₂, R₃, R'₃, R₄, R'₄, R₅, R'₅ sont, indépendamment les uns des autres, choisis parmi un atome d'hydrogène, un atome d'halogène, un radical hydroxyle, un radical alkyle, alkyloxy, acyle ou acyloxy, linéaire ou ramifié, saturé ou insaturé, ayant 1 à 6 atomes de carbone, dans une composition cosmétique, notamment de maquillage, pour lui conférer des propriétés de longue tenue dans le temps et/ou de non-migration et/ou de sans-transfert et/ou de non-formation de stries, en particulier sur les paupières.

L'invention a également pour objet l'utilisation d'au moins un composé indigoïde de formule (I) ci-dessus pour la protection anti-microbienne des compositions cosmétiques, notamment de maquillage.

Il est usuel d'introduire dans les compositions cosmétiques, notamment de maquillage, des liants siliconés afin d'en faciliter l'application et l'étalement tout en procurant du confort et de la douceur.

Cependant, l'inventeur a constaté que l'absence d'huile de silicone non-volatile dans le liant siliconé des compositions de maquillage, a tendance à diminuer la tenue dans le temps de ces compositions, en particulier des fards à paupières. En l'absence d'huile de silicone non-volatile, le fard à paupières se transforme en poudre et ne tient pas.

L'inventeur a découvert, de manière surprenante, qu'en associant des huiles de silicone, en particulier non-volatiles, dans des compositions cosmétiques, notamment de maquillage, à un agent colorant indigoïde de formule (I) ci-dessus, on obtient des compositions cosmétiques ayant non seulement de bonnes propriétés d'étalement, de confort et de douceur, mais également de bonnes propriétés de tenue dans le temps, aussi bien de la couleur que du maquillage.

De plus, les compositions de maquillage ainsi obtenues peuvent conférer une couleur intense, notamment bleue, très saturée et très couvrante, qui ne s'estompe pas au cours du temps. Elles ne migrent pas et ne dégorgent pas sur (ne tachent pas) le support sur lequel elles sont déposées; elles ne transfèrent pas sur un autre support et ne forment pas de stries, en particulier sur les paupières.

L'inventeur a également découvert, de manière surprenante, que l'utilisation d'huiles de silicone volatiles dans le liant siliconé d'une composition de maquillage contenant un colorant indigoïde de formule (I), conférait à cette dernière des propriétés avantageuses de sans-transfert, c'est-à-dire de non migration sur certains supports avec lesquels elle peut entrer en contact, comme un vêtement, un objet solide ou la peau.

La présente invention a donc également pour objet une composition cosmétique, notamment de maquillage, comprenant dans un milieu cosmétiquement acceptable contenant au moins une huile de silicone non-volatile, au moins un composé indigoïde de formule (I) telle que définie ci-dessus.

Les composés utilisés dans la composition cosmétique de l'invention sont, pour certains, des composés connus dans la littérature. Certains ont notamment été décrits dans la publication "2,2'-Binaphthyliden-1,1'-dione, Farbe und Struktur" de Göltner et al., Liebigs Ann. Chem. 1991, pages 1085-1089. Cette publication décrit notamment un procédé de préparation permettant d'obtenir certains de ces composés sous forme de cristaux de couleur mauve ou bleu, allant du bleu pâle au bleu foncé.

Toutefois, cette publication ne laisse nullement envisager que ces composés peuvent être employés avec succès dans des compositions cosmétiques, c'est-à-dire qu'ils permettent l'obtention d'une composition cosmétiquement acceptable, susceptible d'être appliquée sur la peau, donnant une couleur intense, très saturée et très couvrante, ayant une longue tenue dans le temps, ne migrant pas et ne dégorgeant pas sur le support sur lequel elle est déposée, ne transférant pas sur un autre support et ne formant pas de stries, en particulier sur les paupières.

L'inventeur a également constaté qu'il était en outre possible de moduler la couleur des composés de formule (I) en faisant varier la nature et/ou la position des différents substituants R présents sur la molécule.

On peut ainsi obtenir des composés dont la couleur peut varier du mauve au rouge, en passant par le bleu et le vert.

De plus, les composés utilisés dans la composition cosmétique de l'invention présentent une bonne stabilité à la température, au pH et à la lumière.

Ils sont également aisément accessibles par synthèse chimique, même à un niveau industriel.

Dans la formule (I) ci-dessus, les hétéroatomes peuvent être des atomes ou groupements d'atomes d'oxygène, de silicium, d'azote ou de soufre.

De préférence, R₁ et/ou R'₁ sont des radicaux alkyles ayant 1 à 8 atomes de carbone, et notamment des radicaux méthyle, éthyle, propyle, butyle, pentyle ou hexyle.

De préférence, R₂ à R₅ et R'₂ à R'₅ représentent un atome d'hydrogène.

En particulier, on peut citer comme composés susceptibles d'être utilisés selon l'invention, les composés suivants :
- la 4,4'-di-méthyloxy-[2,2'-binaphtylidène]-1,1'-dione,
- la 4,4'-di-éthyloxy-[2,2'-binaphthylidène]-1,1'-dione,
- la 4,4'-di-isopropyloxy-[2,2'-binaphtylidène]-1,1'-dione, et
- la 4,4'-di-n-hexyloxy-[2,2'-binaphtylidène]-1,1'-dione.

La 4,4'-di-méthyloxy-[2,2'-binaphtylidène]-1,1'-dione est préférée pour être utilisée selon l'invention car elle permet d'obtenir une couleur bleue intense de longue tenue dans le temps.

Les composés de formule (I) se présentent sous forme solide. Ils produisent des couleurs vives et variées, selon la nature des substituants.

Ils sont généralement insolubles dans l'eau et très peu solubles dans des huiles de nature et/ou de polarité variées. En conséquence, ces composés présentent l'avantage de très peu migrer lorsqu'ils sont utilisés dans des compositions comprenant des corps gras.

Les composés selon l'invention peuvent être aisément préparés par l'homme du métier sur la base de l'art antérieur et de ses connaissances techniques générales.

Les composés de formule (I) peuvent être incorporés dans une composition cosmétique, notamment de maquillage, en une quantité qui peut être comprise entre 0,5 et 30% en poids par rapport au poids total de la composition, de préférence en une quantité de 0,5 à 10% en poids.

Lesdits composés peuvent être présents dans la composition sous forme libre ou sous forme d'une association avec des particules substrats qu'ils enrobent.

En effet, on a constaté que les composés de formule (I) présentent la particularité de pouvoir enrober, au moins partiellement voire totalement, des particules substrats telles que des pigments ou des charges usuelles.

Parmi les particules susceptibles d'être enrobées par les composés de formule (I), on peut citer notamment les pigments ou nanopigments d'oxydes métalliques comme les oxydes de titane, de zinc, de fer, de manganèse, de cérium et/ou de zirconium; les charges telles que le talc, le mica, la silice, le kaolin, les poudres de Nylon et de polyéthylène; les microsphères telles que les microsphères creuses de copolymères de chlorure de vinylidène/acrylonitrile.

De préférence, on choisit le talc comme particule substrat à enrober.

Les matières pulvérulentes ainsi obtenues, constituées des particules substrats enrobées, peuvent alors elles-mêmes être utilisées comme agent colorant dans les compositions cosmétiques.

On a constaté que lesdites matières pulvérulentes présentent, en microscopie électronique, une structure très homogène.

On a également constaté que lesdites matières pulvérulentes peuvent présenter les propriétés et avantages de chacun des matériaux de départ; en particulier, lorsque l'on enrobe du talc, connu pour apporter de la douceur, on obtient un talc enrobé qui conserve sa douceur.

De plus, l'utilisation d'une très faible quantité de composé de formule (I) enrobant une charge usuelle, permet l'obtention d'un agent colorant ayant une force colorante importante, même utilisé en petite quantité, et apportant une couvrance adéquate ainsi qu'une couleur ayant une bonne tenue à la lumière.

De préférence, on utilisera les composés de formule (I) en une quantité de 0,1 à 100 parties en poids pour 100 parties en poids de particules substrats à enrober.

De préférence, la matière pulvérulente enrobée est constituée de 1 à 20% en poids de composés de formule (I) et de 80 à 99% en poids de particules substrats. Toutefois, il est possible d'envisager une matière pulvérulente enrobée constituée de 50% en poids de composés de formule (I) et de 50% en poids de particules substrats.

D'une manière générale, on préparera la matière pulvérulente constituée de particules substrats au moins partiellement enrobées avec le composé de formule (I), de la manière suivante :
- dans un premier temps, on solubilise le composé de formule (I) dans un solvant adéquat, par exemple le diméthylformamide,
- puis on précipite ledit composé sur la particule substrat à enrober, par exemple en ajoutant la solution dudit composé à une dispersion ou suspension aqueuse de ladite particule.

On peut ensuite filtrer, laver et sécher la matière pulvérulente selon les techniques classiques.

Lesdits composés de formule (I) et/ou lesdites matières pulvérulentes peuvent être utilisés notamment comme agents colorants, dans une composition cosmétique qui peut se présenter sous la forme d'un produit à appliquer sur les lèvres et/ou les tissus kératiniques tels que la peau et les phanères (ongles, cils, sourcils, poils et cheveux).

Ladite composition contient donc un milieu cosmétiquement acceptable, c'est-à-dire un milieu compatible avec toutes les matières kératiniques telles que la peau, les ongles, les cheveux, les cils et sourcils, les lèvres, et toute autre zone cutanée du corps et du visage.

Ledit milieu peut comprendre ou se présenter sous la forme de, notamment, une suspension, une dispersion, une solution en milieu solvant ou hydroalcoolique, éventuellement épaissie voire gélifiée; une émulsion huile-dans-eau, eau-dans-huile, ou multiple; un gel ou une mousse; un gel émulsionné; une dispersion de vésicules notamment lipidiques; une lotion biphase ou multiphase; un spray; une poudre libre, compacte ou coulée; une pâte anhydre.

L'homme du métier pourra choisir la forme galénique appropriée, ainsi que sa méthode de préparation, sur la base de ses connaissances générales, en tenant compte, d'une part de la nature des constituants utilisés, notamment de leur solubilité dans le support, et d'autre part de l'application envisagée pour la composition.

La composition cosmétique selon l'invention comprend une phase grasse contenant au moins une huile de silicone non-volatile, ainsi qu'éventuellement une huile de silicone volatile.

On entend par huile de silicone toute silicone fluide et en particulier liquide à température ambiante et pression atmosphérique, ainsi que toute solution de silicone.

Les huiles de silicone peuvent être des tensio-actifs siliconés ou contenir des tensio-actifs siliconés comme les alkyl, alcoxy ou aryl diméthicone copolyols dont la chaîne alkyle, alcoxy ou aryle contient 1 à 24 atomes de carbone et peut être un radical phényle et en particulier cétyle.

A titre d'exemples d'huiles de silicones utilisées dans l'invention, on peut citer aussi :
- les polyalkylsiloxanes non volatils à chaîne alkyle en C₁-C₂₄ et à groupements terminaux triméthylsilyle, de préférence ceux dont la viscosité à 25°C est inférieure ou égale à 0,06 m²/s, parmi lesquels on peut citer les polydiméthylsiloxanes linéaires, tels que les diméthicones (dénomination CTFA) et notamment ceux vendus sous la dénomination "DOW CORNING FLUID 200" par la Société DOW CORNING, les alkylméthylpolysiloxanes tels que la cétyldiméthicone (dénomination CTFA), ainsi que les produits vendus sous les dénominations "AK" par la société WACKER, "SF" par la société GENERAL ELECTRIC et "ABIL" par la société GOLDSCHMIDT, tels que le produit "ABIL 10" ;
- les silicones non volatiles modifiées par des groupements aliphatiques et/ou aromatiques, éventuellement fluorés, ou par des groupements hydroxyles, thiols et/ou amines;
- les huiles de silicone non volatiles phénylées comme les phényltriméthicones et les phényldiméthicones; on peut citer en particulier celles décrites dans EP-A-665 008 et plus particulièrement les produits vendus sous les dénominations "ABIL AV 8853" par la Société GOLDSCHMIDT, "DC 556" et "SF 558" par la Société DOW CORNING, "SILBIONE 70633 V30" par la Société RHONE POULENC et "BELSIL PDM 100", "BELSIL PDM 200" ou "BELSIL PDM 1000" par la Société WACKER;
- les silicones volatiles à température ambiante (25°C) cycliques ayant de 3 à 8 atomes de silicium et de préférence 4 à 6, comme par exemple les cyclométhicones telles que le cyclotétradiméthylsiloxane, le cyclopentadiméthylsiloxane (D5) ou le cyclohexadiméthylsiloxane (D6), et les produits vendus sous les dénominations : "DC FLUID 244", "DC FLUID 245", "DC FLUID 344" et "DC FLUID 345" par la société DOW CORNING ainsi que ceux vendus sous les dénominations "ABIL K4" par la société GOLDSCHMIDT, sous les dénominations "SILBIONE 70045 V2" et "SILBIONE HUILE 70045 V5" par la société RHONE POULENC ainsi que sous les dénominations "VOLATILE SILICONE 7158" et "VOLATILE SILICONE 7207" par la société UNION CARBIDE ;
- les cyclocopolymères non volatils du type diméthylsiloxane/ méthylalkylsiloxane, tels que la "silicone FZ 3109", vendue par la Société UNION CARBIDE, qui est un cyclocopolymère diméthyl-siloxane/méthyloctylsiloxane;
- les silicones volatiles linéaires ayant de 2 à 9 atomes de silicium, comportant éventuellement une chaîne alkyle pendante ou en bout de chaîne en C₂-C₁₀, par exemple l'hexaméthyldisiloxane, l'hexylheptaméthyltrisiloxane et l'octylheptaméthyltrisiloxane;
et leurs mélanges.

Les huiles de silicone non volatiles peuvent être aussi des résines liquides comme les isostéaryl triméthylolpropane siloxy silicates.

Les huiles de silicone utilisées selon l'invention sont de préférence présentes dans une proportion d'au moins 0,5% et de préférence d'au moins 4% en poids, par rapport au poids total de la composition. Dans le cas d'un fard à paupières, la quantité d'huile de silicone peut aller jusqu'à 70% du poids total de la composition, et représenter en particulier de 4 à 40% en poids du poids total de la composition.

La composition selon l'invention peut également comprendre d'autres composés siliconés tels que les gommes de silicone et les cires de silicone.

Les gommes de silicone utilisables dans la composition de l'invention peuvent être des polysiloxanes de masse moléculaire élevée, de l'ordre de 200 000 à 1 000 000 et de viscosité supérieure à 500 000 mPa.s. Elles peuvent être utilisées seules ou en mélange avec un solvant tel qu'une huile polydiméthylsiloxane ou polyphénylsiloxane, ou une cyclométhicone.

Les cires de silicone utilisables dans la composition selon l'invention peuvent être des polysiloxanes linéaires substitués. On peut citer, par exemple, les cires de silicone polyéther, les alkyl ou alkoxydiméthicones ayant de 16 à 45 atomes de carbone.

Les compositions selon l'invention peuvent également comprendre des corps gras non siliconés dont des corps gras pâteux, des gommes, des cires et des huiles d'origine végétale, minérale, animale ou synthétique.

Par cire, on entend un composé solide à température ambiante, généralement cristallin et ayant un point de fusion supérieur à 45°C.

On peut définir les composés gras pâteux à l'aide d'au moins l'une des propriétés physico-chimiques suivantes :
- une viscosité de 0,1 à 40 Pa.s (1 à 400 poises), mesurée à 40°C avec un viscosimètre rotatif CONTRAVES TV équipé d'un mobile MS-r3 ou MS-r4 à la fréquence de 60 Hz,
- un point de fusion de 25-70°C, de préférence 25-55°C.

Comme cires utilisables dans l'invention, on peut citer les cires d'origine animale comme la lanoline, la cire d'abeilles, le spermaceti, les dérivés de la lanoline tels que les alcools de lanoline, la lanoline hydrogénée, hydroxylée ou acétylée, les acides gras de la lanoline et l'alcool de lanoline acétylée ; les cires d'origine végétale telles que la cire de Carnauba, de Candellila, de kapok, d'Ouricury, de riz, de jojoba hydrogénée, d'Alfa, du Japon ou les cires de fibres de liège ou de canne à sucre ou encore le beurre de cacao ; les cires minérales par exemple de paraffine, de montan, de lignite, de pétrolatum, de vaseline ou les cires microcristallines, la cérésine, l'ozokérite ; les cires synthétiques comme les cires de polyéthylène, les cires obtenues par synthèse de Fischer-Tropsch et les esters linéaires résultant de la réaction d'un acide carboxylique saturé en C₁₀ à C₄₀ et d'un alcool saturé en C₁₀ à C₄₀ comme le myristate de myristyle. On peut aussi utiliser les lanolates ou stéarates de calcium et l'huile de coprah ou de jojoba hydrogénée.

La phase grasse peut aussi comprendre une ou des huile(s) hydrocarbonée(s) ou fluorée(s).

Comme huile hydrocarbonée, on citera : toute huile (ou mélange d'huiles) fluide, stable à la température d'utilisation habituelle des produits cosmétiques, pharmaceutiques, hygiéniques, telle que les huiles d'origine végétale ou animale, minérale ou synthétique, les triglycérides d'acides gras en C₁₂ à C₁₈.

Parmi les huiles d'origine végétale ou animale, modifiées ou non, on peut citer par exemple l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum, le perhydrosqualène.

Parmi les huiles d'origine minérale, on peut citer par exemple l'huile de paraffine et l'huile de vaseline.

Parmi les huiles synthétiques, on peut citer notamment les esters d'acides gras comme le myristate d'isopropyle, le palmitate d'isopropyle, le palmitate de 2-éthylhexyle, l'huile de Purcellin (octanoate de stéaryle), l'isononanoate d'isononyle ou d'isostéaryle, le lanolate d'isopropyle, les acides gras, comme les acides oléique, palmitique, stéarique, béhénique, linoléique et linolénique, les isoparaffines volatiles ou non comme les isoparaffines en C₈-C₁₆ et les polyisobutènes.

Lorsque la composition se présente sous forme aqueuse, notamment sous forme de dispersion ou d'émulsion, elle peut comprendre une phase aqueuse, qui peut comprendre de l'eau, une eau florale et/ou une eau minérale.

Ladite phase aqueuse peut comprendre de 0,5 à 20% en poids, par rapport au poids total de la phase aqueuse, d'un monoalcool inférieur en C₂-C₆ et/ou d'un polyol tel que le glycérol, le butylèneglycol, l'isoprène glycol, le propylèneglycol, le polyéthylèneglycol.

Lorsque la composition selon l'invention se présente sous la forme d'une émulsion, elle peut éventuellement comprendre en outre un tensio-actif, de préférence en une quantité de 0,01 à 30% en poids par rapport au poids total de la composition.

La composition selon l'invention peut également comprendre de 0,05 à 15% en poids, par rapport au poids total de l'émulsion, d'au moins un co-émulsionnant qui peut être choisi parmi le monostéarate de sorbitan oxyéthyléné, des alcools gras tels que l'alcool stéarylique ou l'alcool cétylique, ou des esters d'acides gras et de polyols tels que le stéarate de glycéryle.

La composition selon l'invention peut encore comprendre un ou plusieurs agents épaississants dans des concentrations allant jusqu'à 10% en poids, par rapport au poids total de la composition, choisis parmi :
- les biopolymères polysaccharides comme la gomme de xanthane, la gomme de caroube, la gomme de guar, les alginates, les celluloses modifiées, les dérivés de l'amidon, les dérivés d'éthers de cellulose possédant des groupes ammonium quaternaires, les polysaccharides cationiques;
- les polymères synthétiques comme les acides polyacryliques, la polyvinylpyrrolidone, l'alcool polyvinylique, les polymères à base de polyacrylamide;
- le silicate de magnésium et d'aluminium.

Selon l'application envisagée, la composition peut comprendre en outre un polymère filmogène. Ceci est notamment le cas lorsque l'on souhaite préparer une composition de type vernis à ongles, mascara, eye-liner ou composition capillaire de type laque. Les polymères peuvent être dissous ou dispersés dans le milieu cosmétiquement acceptable. En particulier, le polymère peut être présent sous forme de solution dans un solvant organique ou sous forme de dispersion aqueuse de particules de polymère filmogène. Ledit polymère peut être choisi parmi la nitrocellulose, l'acétobutyrate de cellulose, les butyralpolyvinyliques, les résines alkydes, les polyesters, les acryliques, les vinyliques et/ou les polyuréthannes.

La composition peut également comprendre au moins un plastifiant, qui peut être présent à une teneur allant de 1 % à 40% en poids par rapport au poids total de la composition.

La composition peut comprendre en outre une phase particulaire, qui peut comprendre des pigments et/ou des nacres et/ou des charges habituellement utilisés dans les compositions cosmétiques. Par pigments, il faut comprendre des particules blanches ou colorées, minérales ou organiques, destinées à colorer et/ou opacifier la composition.

Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage.

Par nacres, il faut comprendre des particules irisées qui réfléchissent la lumière.

Les pigments peuvent être présents dans la composition à raison de 0 à 50% en poids de la composition finale, et de préférence à raison de 2 à 30% en poids. Ils peuvent être blancs ou colorés, minéraux et/ou organiques, de taille usuelle ou nanométrique. On peut citer les dioxydes de titane, de zirconium ou de cérium, ainsi que les oxydes de zinc, de fer ou de chrome, le bleu ferrique, l'hydrate de chrome, le noir de carbone, les outremers (polysulfures d'aluminosilicates), le pyrophosphate de manganèse et certaines poudres métalliques telles que celles d'argent ou d'aluminium. On peut encore citer les laques couramment employées pour conférer aux lèvres et à la peau un effet de maquillage, qui sont des sels de calcium, de baryum, d'aluminium ou de zirconium, de colorants acides.

Les nacres peuvent être présentes dans la composition à raison de 0 à 50% en poids, de préférence à un taux de l'ordre de 1 à 25% en poids. Parmi les nacres envisageables, on peut citer la nacre naturelle, le mica recouvert d'oxyde de titane, d'oxyde de fer ou de pigment naturel, l'oxychlorure de bismuth, ainsi que le mica titane coloré.

Les charges, qui peuvent être présentes à raison de 0 à 80% en poids, de préférence 1 à 60%, dans la composition, peuvent être minérales ou de synthèse, lamellaires ou non lamellaires. On peut citer le talc, le mica, la silice, le kaolin, les poudres de Nylon et de polyéthylène, le Téflon, l'amidon, le nitrure de bore, les microsphères de polymère telles que l'Expancel (Nobel Industrie), le polytrap (Dow Corning) et les microbilles de résine de silicone (Tospearls de Toshiba, par exemple), le carbonate de calcium précipité, le carbonate ou l'hydrocarbonate de magnésium, des savons métalliques dérivés d'acides organiques carboxyliques ayant de 8 à 22 atomes de carbone.

Selon le type de formulation, la phase pulvérulente peut représenter de 0,01 à 99,5% en poids de la composition.

La composition peut en outre comprendre un colorant, notamment un colorant organique naturel tel que le carmin de cochenille, et/ou un colorant de synthèse tel que les colorants halogéno-acides, azoïques, anthraquinoniques. On peut également citer des colorants minéraux tels que le sulfate de cuivre.

La composition peut comprendre en outre tout additif usuellement utilisé dans le domaine cosmétique, tel que des antioxydants, des parfums, des huiles essentielles, des conservateurs, des actifs cosmétiques ou dermatologiques lipophiles ou hydrophiles comme les hydratants, les vitamines, les sphingolipides, les agents autobronzants tels que la DHA, les filtres solaires, des agents antimousses, des agents séquestrants, des émollients.

Bien entendu, l'homme du métier veillera à choisir les éventuels composés complémentaires, et/ou leur quantité, de manière telle que les propriétés avantageuses de la composition selon l'invention ne soient pas, ou substantiellement pas, altérées par l'adjonction envisagée.

Les compositions cosmétiques selon l'invention peuvent se présenter sous la forme d'un produit de soin et/ou de maquillage de la peau, un produit solaire ou autobronzant, un produit capillaire. Elles trouvent une application particulière dans le domaine du maquillage, notamment comme rouges à lèvres, fonds de teint, fards à joues ou à paupières, poudres libres ou compactes, eye-liners, mascaras ou vernis à ongles.

L'invention est illustrée plus en détail dans les exemples suivants.

### Exemple 1 : Préparation de la 4,4'-di-méthyloxy-[2,2'-binaphthylidène]-1,1'-dione

On dissout, à température ambiante, 10 g de 4-méthoxy-1-naphtol dans un mélange de 1 litre de diméthylacétamide, 250 ml de méthanol et 150 ml d'eau.

On ajoute, sous agitation, 31 g (2 équivalents) de chlorure ferrique hexahydraté.

On laisse agiter pendant 5 minutes, puis on filtre le précipité bleu sur verre fritté.

On lave et sèche le composé de manière usuelle.

On obtient 5,2 g (rendement : 52%) du pigment recherché, sous forme d'une poudre amorphe bleu/violet.
Point de fusion : 274°C
HPCCM(CH₂Cl₂): profil monotache Rf = 0,7
HPLC : profil monopic
Spectres de masse, RMN et UV : conformes à la structure attendue.

| Analyse élémentaire : | | | |
|---|---|---|---|
| | C% | H% | 0% |
| Théorique | 76,73 | 4,68 | 18,20 |
| Expérimental | 76,94 | 4,64 | 18,53 |

On teste la stabilité de ce composé et l'on obtient les résultats suivants :
- stabilité à 90°C : au moins 16 heures
- stabilité à 45°C : au moins 1 mois
- stabilité à pH 4 : au moins 1 mois
- stabilité à pH 10 : au moins 1 mois
- stabilité à la lumière (Suntest) : au moins 36 heures.

### Exemple 2 : Préparation de la 4,4'-di-éthyloxy-[2,2'-binaphthylidène]-1,1'-dione

On dissout, à température ambiante, 20 g de 4-éthoxy-1-naphtol dans 500 ml de chloroforme.

On ajoute, sous agitation, 19 g d'oxyde d'argent et on laisse agiter pendant une heure.

On filtre le précipité sur verre fritté, on le lave avec du dichlorométhane bouillant jusqu'à décoloration du solvant, on concentre la phase organique et on obtient des cristaux de couleur violet foncé, qui sont séchés.

On obtient 10 g (rendement : 50%) du pigment recherché, sous forme de cristaux.
Point de fusion : 244°C
HPCCM (CH₂Cl₂) : profil monotache Rf = 0,8
Spectre RMN : conforme à la structure attendue.

| Analyse élémentaire : | | | |
|---|---|---|---|
| | C% | H% | 0% |
| Théorique | 77,40 | 5,41 | 17,18 |
| Expérimental | 77,46 | 5,30 | 17,03 |

### Exemple 3 : Préparation de la 4,4'-di-isopropyloxy-[2,2'-binaphthylidène]-1,1'-dione

On procède de manière similaire à l'exemple 2, à partir de 20 g de 4-isopropyl-1-naphtol et de 20 g d'oxyde d'argent.

On obtient 16,4 g de cristaux violet (rendement : 83%).
Point de fusion : 230°C
HPCCM (dichlorométhane 8/heptane 2) : profil monotache
Rf = 0,5
Spectre RMN : conforme à la structure attendue.

| Analyse élémentaire : | | | |
|---|---|---|---|
| | C% | H% | 0% |
| Théorique | 77,98 | 6,04 | 15,98 |
| Expérimental | 78,41 | 6,10 | 15,58 |

### Exemple 4 : Préparation de la 4,4'-di-n-hexyloxy-[2,2'-binaphthylidène]-1,1'-dione

On procède de manière similaire à l'exemple 2, à partir de 7 g de 4-n-hexyl-1-naphtol et de 14 g d'oxyde d'argent.

On obtient 6,0 g de cristaux violet (rendement : 86%).
Point de fusion : 146°C
HPCCM (dichlorométhane 4/heptane 6) : profil monotache
Rf = 0,2
Spectre RMN : conforme à la structure attendue.

### Exemple 5 : enrobage de talc

On dissout à chaud 0,6 g de 4,4'-di-méthyloxy-[2,2'-binaphthylidène]-1,1'-dione dans 100 ml de diméthylformamide.

On coule lentement ce mélange sur une suspension vivement agitée de 20 g de talc dans 200 ml d'eau.

On laisse refroidir jusqu'à température ambiante, on filtre sur verre fritté, on lave à l'eau et on sèche.

On obtient un pigment de couleur homogène bleu clair, supporté à 3% en poids sur le talc.

### Exemple 6

On dissout à chaud 2,05 g de 4,4'-di-méthyloxy-[2,2'-binaphthylidène]-1,1'-dione dans 410 ml de diméthylformamide.

On coule lentement ce mélange sur une suspension vivement agitée de 20,5 g de talc dans 820 ml d'eau.

On laisse refroidir jusqu'à température ambiante, on filtre sur verre fritté, on lave à l'eau et on sèche.

On obtient un pigment de couleur homogène bleue, supporté à 10% en poids sur le talc.

### Exemple 7

On dissout à chaud 4,1 g de 4,4'-di-méthyloxy-[2,2'-binaphthylidène]-1,1'-dione dans 820 ml de diméthylformamide.

On coule lentement ce mélange sur une suspension vivement agitée de 20,5 g de talc dans 1640 ml d'eau.

On laisse refroidir jusqu'à température ambiante, on filtre sur verre fritté, on lave à l'eau et on sèche.

On obtient un pigment de couleur homogène bleu foncé, supporté à 20% en poids sur le talc.

### Exemple 8

On prépare un fard à paupières comprenant les ingrédients suivants :
- Triisocétyl citrate 0,275 g
- 4,4'-diméthyloxy-[2,2'-binaphthylidène]-1,1'-dione (composé de l'exemple 1) 5 g
- Dioxyde de titane 2 g
- Oxychlorure de bismuth 20 g
- Mica 5 g
- Talc 48,3 g
- Stéarate de zinc 4 g
- Poly(méthyl méthacrylate) réticulé 10 g
- Diméthicone 3,4595 g
- Diméthicone triméthylsiloxysilicate 1,089 g
- Cétyl diméthicone 0,6765 g
- Parahydroxybenzoate de méthyle 0,2 g

On obtient un fard à paupières de couleur bleue intense, de très forte couvrance et ayant une très bonne tenue sur les paupières.

La couleur reste homogène et ne s'estompe pas dans le temps.

En outre, le fard à paupières ne migre pas, ne dégorge pas et ne forme pas de stries sur les paupières après 4 heures.

### Exemple 9 (comparatif)

On prépare un fard à paupières à partir des ingrédients suivants :
- Triisocétyl citrate 0,275 g
- Violet de manganèse 14 g
- Oxydes de fer 2 g
- Bleu d'Outremer et silice 25 g
- Carmin de cochenille 3 g
- Dioxyde de titane 2 g
- Oxychlorure de bismuth 20 g
- Mica 5 g
- Talc 9,3 g
- Stéarate de zinc 4 g
- Poly(méthyl méthacrylate) réticulé 10 g
- Diméthicone 3,4595 g
- Diméthicone triméthylsiloxysilicate 1,089 g
- Cétyl diméthicone 0,6765 g
- Parahydroxybenzoate de méthyle 0,2 g

On obtient un fard à paupières de couleur bleue très proche de celle obtenue avec le fard à paupières de l'exemple 8, selon l'invention.

Néanmoins, ce fard à paupières est moins couvrant.

En outre, les tests de tenue dans le temps effectués sur 6 modèles montrent une moins bonne tenue avec une perte d'intensité pour 1 modèle, une teinte non uniforme ou qui s'estompe pour 2 modèles et qui disparaît pour 1 modèle.

La présence de stries sur les paupières est observée pour 2 modèles.

Les résultats des tests de tenue dans le temps sur 6 modèles après 4 heures sont les suivants :

| Tenue | Fard à paupières de l'exemple 8 (invention) | Fard à paupières de l'exemple 9 (comparatif) |
|---|---|---|
| Très bonne | 2 | - |
| Bonne | 3 | 3 |
| | | mais perte d'intensité (1) |
| Moyenne | 1 | 2 |
| | perte d'intensité | non uniforme (1) |
| | | très estompé (1) |
| Mauvaise | - | 1 |
| | | la couleur a disparu |
| Présence de stries : - oui | 1 | 2 |
| - non | 5 | 4 |

### Exemple 10 (tests microbiologiques)

Le pigment de l'exemple 1, à savoir la 4,4'-diméthyloxy-[2,2'-binaphthylidène]-1,1'-dione, a été comparé au violet de manganèse.

On filtre des spores de moisissures d'Aspergillus niger et Penicillium chrysogenum. On les met en suspension dans un diluant. On filtre et on dépose le filtre à la surface du pigment sous forme de poudre. On dénombre ensuite le nombre de germes à la surface de chaque pigment et on évalue le développement macroscopique des moisissures après incubation à 30°C et 95% d'humidité relative pendant 21 jours.

Les résultats sont les suivants :

| Germes | Inoculum (germes/filtre) | Vieillissement à 30°C (21 jours) | |
|---|---|---|---|
| | | Pigment selon l'invention | Violet de manganèse |
| Aspergillus niger | 7,0.10³ | 3,4.10⁴ | 7,0.10⁵ |
| Penicillium chrysogenum | 1,0.10⁴ | 4,8.10³ | 1,0.10⁶ |

Après 21 jours à 30°C, on ne voit aucune manifestation macroscopique sur le pigment selon l'invention, alors que le violet de manganèse est complètement envahi de moisissures, ce que l'on voit à l'oeil nu; le développement macroscopique est intense.

## Revendications

1. Utilisation d'au moins un composé indigoïde, de formule : 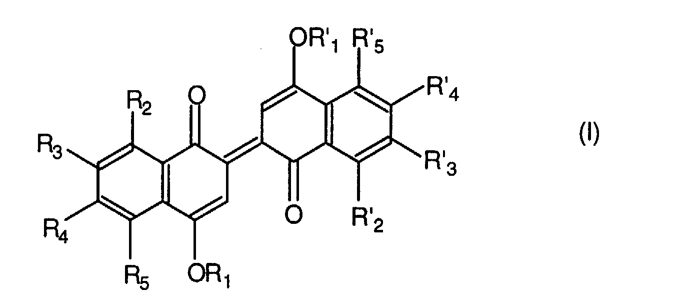 dans laquelle :
- R₁ et R'₁ sont, indépendamment l'un de l'autre, des radicaux alkyles, linéaires, ramifiés ou cycliques, saturés ou insaturés, ayant 1 à 18 atomes de carbone, et éventuellement substitués par un ou plusieurs halogènes, et/ou par un ou plusieurs radicaux hydroxyles, et/ou interrompus par un ou plusieurs hétéroatomes choisis parmi les atomes ou groupements d'atomes d'oxygène, de soufre, d'azote et de silicium;
- R₂, R'₂, R₃, R'₃, R₄, R'₄, R₅, R'₅ sont, indépendamment les uns des autres, choisis parmi un atome d'hydrogène, un atome d'halogène, un radical hydroxyle, un radical alkyle, alkyloxy, acyle ou acyloxy, linéaire ou ramifié, saturé ou insaturé, ayant 1 à 6 atomes de carbone,
dans une composition cosmétique, notamment de maquillage, pour lui conférer des propriétés de longue tenue dans le temps et/ou de non-migration et/ou de sans-transfert et/ou de non-formation de stries.

2. Utilisation d'au moins un composé indigoïde, de formule : 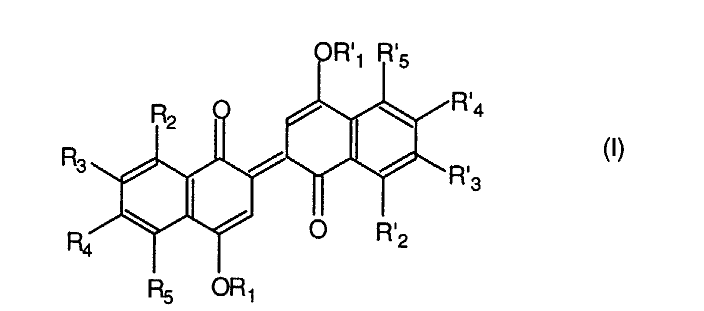 dans laquelle :
- R₁ et R'₁ sont, indépendamment l'un de l'autre, des radicaux alkyles, linéaires, ramifiés ou cycliques, saturés ou insaturés, ayant 1 à 18 atomes de carbone, et éventuellement substitués par un ou plusieurs halogènes, et/ou par un ou plusieurs radicaux hydroxyles, et/ou interrompus par un ou plusieurs hétéroatomes choisis parmi les atomes ou groupements d'atomes d'oxygène, de soufre, d'azote et de silicium;
- R₂, R'₂, R₃, R'₃, R₄, R'₄, R₅, R'₅ sont, indépendamment les uns des autres, choisis parmi un atome d'hydrogène, un atome d'halogène, un radical hydroxyle, un radical alkyle, alkyloxy, acyle ou acyloxy, linéaire ou ramifié, saturé ou insaturé, ayant 1 à 6 atomes de carbone,
pour la protection anti-microbienne d'une composition cosmétique, notamment de maquillage.

3. Utilisation selon la revendication 1 ou 2, caractérisée par le fait que dans la formule (I), les radicaux R₁ et/ou R'₁ sont des radicaux alkyle ayant 1 à 8 atomes de carbone.

4. Utilisation selon l'une quelconque des revendications 1 à 3, caractérisée par le fait que dans la formule (I), les radicaux R₂ à R₅ et R'₂ à R'₅ représentent un atome d'hydrogène.

5. Utilisation selon l'une quelconque des revendications précédentes, caractérisée par le fait que le composé de formule (I) est choisi parmi :
- la 4,4'-di-méthyloxy-[2,2'-binaphtylidène]-1,1'-dione,
- la 4,4'-di-éthyloxy-[2,2'-binaphthylidène]-1,1'-dione,
- la 4,4'-di-isopropyloxy-[2,2'-binaphtylidène]-1,1'-dione, et
- la 4,4'-di-n-hexyloxy-[2,2'-binaphtylidène]-1,1'-dione.

6. Utilisation selon la revendication 5, caractérisée par le fait que le composé de formule (I) est la 4,4'-di-méthyloxy-[2,2'-binaphtylidène]-1,1'-dione.

7. Utilisation selon l'une quelconque des revendications précédentes, caractérisée par le fait que le composé de formule (I) est présent à raison de 0,5 à 30% en poids, de préférence 0,5 à 10% en poids, par rapport au poids total de la composition.

8. Utilisation selon l'une quelconque des revendications précédentes, caractérisée par le fait que le composé de formule (I) est présent sous forme libre ou sous forme d'une association avec des particules substrats qu'il enrobe.

9. Utilisation selon la revendication 8, caractérisée par le fait que les particules substrats sont choises parmi les pigments ou nanopigments d'oxydes métalliques, les charges telles que le talc, le mica, la silice, le kaolin, les poudres de Nylon et de polyéthylène et les microsphères telles que les microsphères creuses de copolymères de chlorure de vinylidène/acrylonitrile.

10. Composition cosmétique, notamment de maquillage, comprenant, dans un milieu cosmétiquement acceptable contenant au moins une huile de silicone non-volatile, au moins un composé indigoïde de formule : 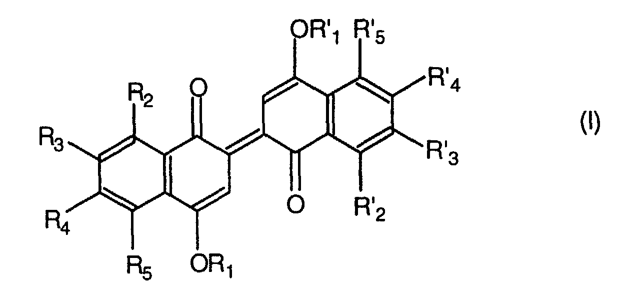 dans laquelle :
- R₁ et R'₁ sont, indépendamment l'un de l'autre, des radicaux alkyles, linéaires, ramifiés ou cycliques, saturés ou insaturés, ayant 1 à 18 atomes de carbone, et éventuellement substitués par un ou plusieurs halogènes, et/ou par un ou plusieurs radicaux hydroxyles, et/ou interrompus par un ou plusieurs hétéroatomes choisis parmi les atomes ou groupements d'atomes d'oxygène, de soufre, d'azote et de silicium;
- R₂, R'₂, R₃, R'₃, R₄, R'₄, R₅, R'₅ sont, indépendamment les uns des autres, choisis parmi un atome d'hydrogène, un atome d'halogène, un radical hydroxyle, un radical alkyle, alkyloxy, acyle ou acyloxy, linéaire ou ramifié, saturé ou insaturé, ayant 1 à 6 atomes de carbone.

11. Composition cosmétique selon la revendication 10, caractérisée par le fait que dans la formule (I), les radicaux R₁ et/ou R'₁ sont des radicaux alkyle ayant 1 à 8 atomes de carbone.

12. Composition cosmétique selon la revendication 10 ou 11, caractérisée par le fait que dans la formule (I), les radicaux R₂ à R₅ et R'₂ à R'₅ représentent un atome d'hydrogène.

13. Composition cosmétique selon l'une quelconque des revendications 10 à 12, caractérisée par le fait qu'elle contient au moins un composé de formule (I), choisi parmi :
- la 4,4'-di-méthyloxy-[2,2'-binaphtylidène]-1,1'-dione,
- la 4,4'-di-éthyloxy-[2,2'-binaphthylidène]-1,1'-dione,
- la 4,4'-di-isopropyloxy-[2,2'-binaphtylidène]-1,1'-dione, et
- la 4,4'-di-n-hexyloxy-[2,2'-binaphtylidène]-1,1'-dione.

14. Composition cosmétique selon la revendication 13, caractérisée par le fait qu'elle contient la 4,4'-di-méthyloxy-[2,2'-binaphtylidène]-1,1'-dione.

15. Composition cosmétique selon l'une quelconque des revendications 10 à 14, caractérisée par le fait qu'elle contient 0,5 à 30% en poids, et de préférence 0,5 à 10% en poids d'au moins un composé de formule (I), par rapport au poids total de la composition.

16. Composition cosmétique selon l'une quelconque des revendications 10 à 15, caractérisée par le fait que le composé de formule (I) est présent sous forme libre ou sous forme d'une association avec des particules substrats qu'il enrobe.

17. Composition cosmétique selon la revendication 16, caractérisée par le fait que les particules substrats sont choisies parmi les pigments ou nanopigments d'oxydes métalliques, les charges telles que le talc, le mica, la silice, le kaolin, les poudres de Nylon et de polyéthylène et les microsphères telles que les microsphères creuses de copolymères de chlorure de vinylidène/acrylonitrile.

18. Composition cosmétique selon l'une quelconque des revendications 10 à 17, caractérisée par le fait qu'elle comprend au moins 0,5 % en poids, et de préférence au moins 4% en poids, par rapport au poids total de la composition, d'au moins une huile de silicone.

19. Composition cosmétique selon la revendication 18, caractérisée par le fait que l'huile de silicone est une huile de silicone non-volatile choisie parmi les polyalkylsiloxanes à chaîne alkyle en C₁-C₂₄ et à groupements terminaux triméthylsilyle, tels que les polydiméthylsiloxanes linéaires comme les diméthicones et les alkylméthylpolysiloxanes comme la cétyldiméthicone, les alkyl, alcoxy ou aryl diméthicone copolyols à chaîne alkyle, alcoxy ou aryle en C₁-C₂₄ telle que phényle ou cétyle, les silicones modifiées par des groupements aliphatiques et/ou aromatiques, éventuellement fluorés, ou par des groupements hydroxyles, thiols et/ou amines, les huiles de silicones phénylées comme les phényltriméthicones et les phényldiméthicones et leurs mélanges.

20. Composition cosmétique selon la revendication 19, caractérisée par le fait que l'huile de silicone est choisie parmi les diméthicones et/ou les alkyldiméthicones.

21. Composition cosmétique selon l'une quelconque des revendications 18 à 20, caractérisée par le fait qu'elle contient une huile de silicone volatile.

22. Composition cosmétique selon l'une quelconque des revendications 10 à 21, caractérisée par le fait qu'elle comprend en outre des corps gras non-siliconés choisis parmi les corps gras pâteux, les gommes, les cires et les huiles d'origine végétale, minérale, animale ou synthétique.

23. Composition cosmétique selon l'une quelconque des revendications 10 à 22, caractérisée par le fait qu'elle comprend en outre des pigments ou nanopigments, des nacres et/ou des charges.

24. Composition cosmétique selon l'une quelconque des revendications 10 à 23, caractérisée par le fait qu'elle comprend en outre des additifs choisis parmi les antioxydants, les parfums, les huiles essentielles, les conservateurs, les actifs cosmétiques ou dermatologiques, les agents antimousses, les agents séquestrants, les émollients, les polymères liposolubles et les dispersions de particules de polymères filmogènes dans un milieu aqueux.

25. Composition cosmétique selon l'une quelconque des revendications 10 à 24, caractérisée par le fait qu'elle se présente sous la forme d'une suspension, d'une dispersion, d'une solution en milieu solvant ou hydroalcoolique, éventuellement épaissie ou gélifiée, d'une émulsion huile-dans-eau, eau-dans-huile ou multiple, d'un gel ou d'une mousse, d'un gel émulsionné, d'une dispersion de vésicules notamment lipidique, d'une lotion biphase ou multiphase, d'un spray, d'une poudre libre, compacte ou coulée ou d'une pâte anhydre.

26. Composition cosmétique selon l'une quelconque des revendications 10 à 25, caractérisée par le fait qu'elle se présente sous la forme d'un produit de maquillage de la peau, des lèvres ou des phanères.

27. Composition cosmétique selon l'une quelconque des revendications 10 à 26, caractérisée par le fait qu'elle se présente sous la forme d'un fard à paupières, d'un fard à joues, d'un fond de teint ou d'une poudre libre ou compacte pour le visage.
